# EUROPEAN PATENT SPECIFICATION

(11) **EP 2 554 177 B1**
(45) Date of publication and mention of the grant of the patent: **27.04.2016**
(21) Application number: 11762282.9
(22) Date of filing: 30.03.2011
(51) Int. Cl.: A61K 36/185, A61K 31/025, A61K 36/00, A61K 36/05, A61K 36/53, A61K 36/73, A61K 36/75, A61P 25/00, A61P 43/00, A61K 36/14, A61K 36/15, A61K 36/324

(54) **Cosmetic use of Elemi oil for improving rough skin resulting from the malfunction of the circadian rhythm**
Kosmetische Verwendung von Elemiöl zur Verbesserung von rauher Haut aufgrund einer Fehlfunktion des circadianen Rythmus
Utilisation cosmétique d'huile d'Elemi pour le traitement de la peau rugueuse résultant d'une malfonction du rythme circadien

(30) Priority: 31.03.2010 JP 2010083016
(43) Date of publication of application: 06.02.2013
(73) Proprietor: SHISEIDO COMPANY, LTD., Tokyo 104-8010 (JP)
(72) Inventor: GOZU, Yoko, Yokohama-shi Kanagawa 224-8558 (JP); HAZE, Shinichiro, Yokohama-shi Kanagawa 224-8558 (JP); MORI, Keiko, Yokohama-shi Kanagawa 224-8558 (JP); UMISHIO, Kenichi, Yokohama-shi Kanagawa 224-8558 (JP)
(74) Representative: Ter Meer Steinmeister & Partner
(86) International application number: PCT/JP2011/001940
(87) International publication number: WO 2011/122040

(56) References cited:
- FR-A1- 2 916 633
- JP-A- 62 161 311
- JP-A- 2004 204 421
- JP-A- 2008 266 319
- JEROME SARRIS ET AL: "Herbal medicine for depression, anxiety and insomnia: A review of psychopharmacology and clinical evidence", EUROPEAN NEUROPSYCHOPHARMACOLOGY, ELSEVIER SCIENCE PUBLISHERS BV, AMSTERDAM, NL, vol. 21, no. 12, 6 April 2011 (2011-04-06) , pages 841-860, XP028119196, ISSN: 0924-977X, DOI: 10.1016/J.EURONEURO.2011.04.002 [retrieved on 2011-04-14]
- HAJI S ET AL: "Collagen production promoter for use in skin cosmetics as antiaging agent, comprises essential oil obtained from steam distillation of plants e.g. Sambucus nigra, Canarium luzonicum and Thymus", WPI / THOMSON,, vol. 2006, no. 71, 7 September 2006 (2006-09-07), XP002559981,
- SATOKO WATANABE ET AL. DAITAI IRYO O KAGAKU SURU 8 AROMA THERAPY, ANTI-AGEING IGAKU vol. 5, no. 3, 2009, pages 375 - 379
- YASUKO IGUCHI ET AL.: 'Aroma Therapy ga Shin'ya Kinmu Mae no Suimin Donyu Shoyo Jikan ni Oyobosu Koka Hinoki no Essence o Shiyo shite' TOTTORI SEKIJUJI BYOIN IGAKU ZASSHI vol. 11, 2002, pages 40 - 41

## Description

### Field of the Invention

The present invention relates to to the cosmetic use of Elemi oil as an expression modulator for clock gene Bmal and a circadian rhythm modulator comprising it, as disclosed herein.

### Description of the Related Art

Almost all living organisms on the earth have an internal "biological clock" which oscillates autonomously with a cycle of approximately 24 hours. The biological clock causes a biological daily fluctuation called circadian rhythm, which is considered to control diurnal changes of various biological phenomena (activities) including not only sleep-awake cycles of the organisms, but also body temperature, blood pressure, hormonal secretion, metabolism, as well as mental and physical activities, eating and the like. In recent years, it has been pointed out that the disturbance of the circadian rhythm is a pathogenic factor of various psychosomatic symptoms or disease conditions including sleep disorder, skin diseases, lifestyle-related diseases and neuropsychiatric disorders such as depression and the like.

As illustrated in Fig. 1, the biological clock is controlled by a rhythm-generating system comprising a group of genes called "clock genes." In mammals, the core of the molecular mechanism of the clock is a feedback loop composed of the transcriptional promotion/suppression of the genes coding for four proteins, CLOCK, BMAL1, PERIOD and CRYPTOCHROME. The circadian rhythm is generated by pulsation of the feedback loop with a cycle of approximately 24 hours.

While the control center (central clock) of the circadian rhythm is located in the suprachiasmatic nucleus in the hypothalamus, it has become clear that the clock genes are expressed even in liver, kidney, skin and other peripheral tissues where the circadian rhythm is generated by a similar system. The expression of the peripheral clock genes is regulated by signals from the suprachiasmatic nucleus. It has further been established that the expression of the clock genes in the peripheral tissues and cells is controlled directly by signal stimulating factors like glucocorticoids, catecholamine, angiotensin II and others, to generate a physiological rhythm. In recent years, the circadian rhythm of cultured cells such as fibroblastic cells has been examined by inducing the circadian rhythm of the clock gene expression in vitro to synchronize the expression rhythm with the stimulating factors like glucocorticoids, forskolin, serum and the others, and by evaluating the circadian rhythm using their expression as the criteria (Non-patent Documents 1, 2 and 3).

The clock genes directly control expression rhythms of other genes as transcription factors, and indirectly control diurnal expression of many more genes through regulation of hormonal secretion and the like. It has become clear that disruption of expression rhythms of clock genes in organisms causes troubles of body organs or the endocrine system, leading to various diseases including lifestyle-related diseases such as hypertension (Non-patent Document 4). For example, a person with obesity has been reported to show abnormal expression of the clock genes, and associations with depression and cancer have also been reported. In addition, it has been elucidated that the clock genes regulate circadian rhythm of various physiological functions of the skin. In an experiment using normal human skin fibroblastic cells, the type I collagen gene was reported to be expressed in a circadian rhythm with an expression pattern similar to that of the clock gene Period 2 (Non-patent Document 5).

By modulating the expression of clock genes, it is possible to adjust various behavior rhythms and circadian rhythms of physiological functions of living organisms which are controlled by the clock genes. Accordingly, there is a strong need to develop an agent which can modulate the expression of the clock genes.

### [Non-patent Document 1]

H. Okamura, "Clock Genes in Cell Clocks: Roles, Actions, and Mysteries", Journal of Biological Rhythms, Vol. 19, No. 5, pp. 388-399, 2004.

### [Non-patent Document 2]

A. Balsalobre et al., "A Serum Shock Induces Circadian Gene Expression in Mammalian Tissue Culture Cells", Cell, Vol. 93, pp. 929-937, 1998.

### [Non-patent Document 3]

K. Yagita et al., "Molecular Mechanisms of the Biological Clock in Cultured Fibroblasts", Science, Vol. 292, pp. 278-281, 2001.

### [Non-patent Document 4]

M. Hastings et al., "Circadian clocks: regulators of endocrine and metabolic rhythms", Journal of Endocrinology, Vol. 195, pp. 187-198, 2007.

### [Non-patent Document 5]

K. Izumi et al., "Gaijitsu rizumu wo motsu hifuseiriidennshi no tansaku (Analysis of skin physiological genes having Circadian Rhythm expression)", The Molecular Biology Society of Japan, 32nd Annual Meeting, Abstract 2P-0009, 2009.

Haji S. et al relates to a collagen production promoter consisting of essential oil obtained from steam distillation of plants selected from Sambucus nigra, Canarium luzonicum and Thymus.

FR 2 916 333 relates to a cosmetic skin care process, intended to prevent and/or treat at least one sign of skin aging, comprising topically applying to the skin a composition containing at least one active agent capable of stimulating the expression of tensin 1, and also relates to an extract of Elemi or Canarium indicum L. or Canarium luzonicum or common Canarium and a cosmetic composition containing said extract.

### DISCLOSURE OF THE INVENTION

The invention is as defined in the claims.

In view of the above-described circumstances, the object of the present invention is to provide an agent which can modulate the expression of a Bmal gene, which is a core gene of biological clocks.

The inventors have come to achieve the present invention as defined in the claims as they have found that Elemi oil has a property to induce expression rhythms of clock gene Bmal and also to promote its expression.

An expression modulator for a Bmal gene according to the use of the present invention comprises elemi oil. It had heretofore not been known at all that elemi oil can modulate the expression of the clock gene. In the present invention, the modulation of gene expression includes not only promotion of gene expression but also modulation of the rhythm of gene expression (phase or cycle).

The circadian rhythm modulator for use according to the present invention comprises the above-mentioned expression modulator for a Bmal gene. As described above, the clock genes directly or indirectly control the diurnal expression of various genes involved in the function of body organs and in the endocrine system. By modulating the expression of a Bmal gene, which is a core gene of the biological clock, it is possible to regulate various behavioral rhythms of the living organism and circadian rhythms of physiological functions that are under the control of the gene.

Furthermore, the inventors of the present invention have found in experiments using human skin fibroblastic cells that elemi oil with expression modulating activity on the Bmal gene as described above can also modulate the expression of the gene for hyaluronic acid synthetase (Has). Thus, it is considered that elemi oil as the expression modulator of the Bmal gene of the present invention can improve the skin function by regulating the circadian rhythm of the hyaluronic acid production in the skin and by augmenting its production.

The expression modulator of the Bmal gene for use according to the present invention may be used alone or in combination with an expression modulator for other clock genes. For example, it may be used in combination with a modulator of expression of a Period gene, one of the other core clock genes. In the circadian rhythm generating system as illustrated in Fig. 1, BMAL1, the expression product of a Bmal gene, forms a hetero dimer with CLOCK to promote transcription of the Period gene, while the expressed PERIOD (PER) forms a hetero dimer with CRYPTOCHROME (CRY) to suppress BMAL and CLOCK activities, so that a feedback loop is created which oscillates with a period of 24 hours. In mammals, circadian rhythms of Bmal and Period genes beat with a phase shift of approximately 12 hours. Bmal expression increases in the night time, while Period expression increases in the daytime. It is therefore considered that the circadian rhythm may be modulated more efficiently by regulating the core loop of the biological clock through the regulated expression of both Bmal and Period genes.

The expression modulator for the Bmal gene of the present invention is elemi oil. It may be applied by various administration modes such as transdermal, oral and inhaled administrations and may be used in cosmetics. It is possible to improve rough skin as a result of the malfunction of the circadian rhythm, by effectively modulating the expression of the Bmal gene.

Furthermore, elemi oil is considered to improve skin function by modulating the circadian rhythm of hyaluronic acid production in skin as well as by augmenting the amount of its production.

### BRIEF DESCRIPTION OF THE DRAWINGS

Fig. 1 is a schematic diagram showing only the core loop of a circadian rhythm generating system by clock genes.
Fig. 2 is a graph showing induction of circadian rhythms of clock gene expression with cortisol and forskolin in cultured human skin fibroblastic cells.
Fig. 3 is a graph showing regulated expression of the clock gene Bmal1 by test substances in cultured human skin fibroblastic cells.

### BEST MODE FOR CARRYING OUT THE INVENTION

The expression modulator for the Bmal gene for use according to the present invention comprises elemi oil as its active ingredient.

As for the details of elemi oil, please refer to Japan Cosmetic Ingredients Dictionary (Nihon Hann-you Keshouhin Genryou Shu) fourth edition (YAKUJI NIPPO LIMITED).

The above-mentioned elemi oil may be obtained by commonly known techniques, for example, by immersing or heating to reflux the plant material from which the extract is derived with an extraction solvent, followed by filtration and concentration. Any solvent which is normally used for extraction may be employed as the extraction solvent, including water, methanol, ethanol, propylene glycol, 1,3-butyleneglycol, glycerin and other alcohols, hydroalcoholic solvents, chloroform, dichloroethane, tetrachloromethane, acetone, ethylacetate, hexane and other organic solvents alone or in combination. The extracts obtained using the above-mentioned solvents may be used as they are, or alternatively, may be used after removing the impurities using an absorption technique with, for example, ion-exchange resin, or porous polymer (e.g. Amberlite XAD-2) column followed by elution with methanol or ethanol and then concentration. Extracts, for example, with water/ethylacetate and others may be used as well.

Elemi oil is commercially available and is briefly described below.

Elemi oil is an essential oil obtained by steam distillation of resin (oleoresin) seeped from the bark of elemi, a medium to tall tree of the Canarium order of the Burseraceae family.

The modulator of circadian rhythm for use according to the present invention contains elemi oil as an expression modulator for the Bmal gene.

Elemi oil as the expression modulator for the Bmal gene may be used alone or in combination with an agent having an action to modulate expression of other clock genes.

Examples of the other clock genes include Period genes (Period1, Period2, Period3), Clock gene, Cryptochrome gene, albumin site D-binding protein (Dbp) gene, E4BP4 gene, Npas2 gene, and Reverb gene. It is, however, preferable for the expression modulator for the Bmal gene to be used in combination with an expression modulator for Period, Clock and/or Cryptochrome gene(s), other core genes of the biological clock, and especially preferable to use in combination with an expression modulator for a Period gene(s).

The expression modulators for the Period gene include, but are not limited to, herbal medicines such as arnica, nuphar, black tea extract, Zanthoxylum and other extracts; and fragrances such as juniper, cedar, lavender, clove bud, cypress, rose, ylang-ylang, galbanum, petitgrain, pepper, thyme, basil, beta-caryophyllene and other essential oils. It has been confirmed that these herbal medicines or fragrances can induce expression rhythm of a Period gene or promote its expression in cultured skin fibroblastic cells.

Further, the expression modulator for the Bmal gene and the circadian rhythm modulator may be used alone or may be contained in various substances. Depending on the kind of substance, any constituent may supplementarily be included as well as the above-mentioned indispensable ingredient.

For example, when the substance is an external formulation to be applied on the skin, any ingredient which is normally found in such an external formulation may be contained together with the above-mentioned expression modulator for the Bmal gene depending on its dosage form (e.g. liquid formulation, powder formulation, granular powder formulation, aerosolized formulation, solid formulation, gel formulation, patch formulation, suppository formulation, and others) or its product form (i.e. cosmetics). An external formulation to be applied on the skin encompasses compositions to be applied on the skin (including head skin, head hair and nails) in general and includes cosmetics such as skin care products, make-up products, hair care products, face wash products, hair wash products and others. The dosage forms include, but are not limited to, water-based systems, solubilized systems, emulsions, oilbased systems, gels, pastes, ointments, aerosols, water-oil two-phase systems, water-oil-powder three phase systems, and others. As the external formulation to be applied on the skin is a cosmetic, it includes perfumes, eaux de toilet, eaux de cologne, creams, emulsions, foundations, face powders, lip sticks, soaps, shampoos and conditioners, body shampoos, body rinses, body powders, bath soaps, and others.

The expression modulator for the Bmal gene for use according to the present invention may be contained in deodorants and any other cosmetic goods, as long as the present invention produces its effect.

The content of the expression modulator for the Bmal gene for use according to the present invention in a substance is not particularly limited, and may be selected appropriately according to the type and form of the elemi oil, but is for example 0.00001 mass% to 100 mass%, preferably 0.0001 mass% to 50 mass%, and more preferably 0.0001 mass% to 20 mass% of the total mass of the substance.

The specific application of the present invention is the cosmetic use of elemi oil for modulating the circadian rhythm for improving rough skin resuting from the malfunction of the circadian rhythm by inducing expression rhythms of a Bmal gene or promoting expression of a Bmal gene.

### [Examples]

The present invention will be described in detail below with examples, but the present invention is not limited to the examples. Skin fibroblastic cells, epithelial cells, endothelial cells, pigment cells, fat cells, nerve cells and various other types of cells may be used as culture cells. In the examples, however, evaluations were carried out with human skin fibroblastic cells. Because the core system of the clock gene is common to all species of organisms and all types of cells, it is thought that evaluation results from human skin fibroblastic cells should be applicable to other species of organisms and other types of cells. In humans, two genes are known as Bmal genes; Bmal1 and Bmal2. They are thought to behave similarly as they belong to the same gene family. In the examples below, Bmal expression was determined as a representative.

### Examination of Evaluation System for Clock Gene Expression Rhythm Using Cultured Human Skin Fibroblastic Cells

It was confirmed that clock gene expression rhythm can be evaluated in a system using cultured human skin fibroblastic cells.

As the cultured human fibroblastic cells, fibroblastic cells from normal human skin were purchased (Cell Application, Inc.) and used in the experiments. They were inoculated in DMEM medium supplemented with 10% FBS, 20 mM HEPES, Glutamax and antibacterial agents and cultured at 37°C in 5% CO₂. On the 6th day of culture, 50 ng/mL of cortisol or 10µM of forskolin was added, and the samples were harvested at various times after time 0, which was defined as the time immediately after the addition of cortisol or forskolin. RNA was extracted from the cells with a commercially available RNA extraction kit, and the amounts of expression of the intended genes were measured by RT-PCR technology using commercially available PCR primers (Perfect Real Time Primer, Takara Bio). As for clock genes, amounts of expression of Period (represented by Period1) and Bmal1, which are involved in the core system, were determined. Similarly, the amounts of expression of a housekeeping gene RPLP0 were quantified and used as an internal standard to calculate the relative expression of the intended genes to RPLP0.

In living organisms, glucocorticoids such as cortisol are involved in regulating the biological clock in peripheral tissue and the like, and it is thought that blood concentration of cortisol rises when waking in the morning, to reset the biological clock. In cultured cells, individual cells usually keep their rhythm independently. The expression rhythms of clock genes can be synchronized to induce a circadian rhythm by stimulating with a signal stimulation factor such as cortisol or forskolin.

The results are shown in Fig. 2. It was confirmed that expression of Bmal1 and Period1 peaks at about 16 hours and about 2 hours, respectively, after stimulation, and that both genes are expressed recurrently with a circadian rhythm of about a 24 hour cycle.

### Evaluation of Test Substances on the Expression Modulating Effect for a Bmal Gene

The above-mentioned results of the evaluation system using cultured human skin fibroblastic cells demonstrate that Bmal1 shows a circadian rhythm with a peak of gene expression at about 16 hours after the stimulation with a reagent. Test substances were evaluated for the expression modulating effect on a Bmal gene based on the amount of gene expression at 16 hours after stimulation. To confirm the induction of expression rhythm for the Bmal gene, the amount of expression of the Bmal gene at 2 hours after stimulation was also determined.

Fibroblastic cells from normal human skin (Cell Application, Inc.) were inoculated according to a method similar to those used above. Each test substance was added on the 6th day of culture to a final concentration of 100 ppm, and the cells were harvested 2 and 16 hours after stimulation. RNA was extracted from the cells with a commercially available RNA extraction kit. The amounts of expression of the Bmal gene were determined by RT-PCR technology using commercially available PCR primers (Perfect Real Time Primer, Takara Bio).

Furthermore, gene expression was also similarly determined for hyaluronic acid synthetase (HAS) involved in the production of hyaluronic acid which plays an important role in retaining skin moisture. HAS1, HAS2 and HAS3 are known as HAS, and HAS2 was examined as a representative hyaluronic acid synthetase.

The amounts of gene expression of a housekeeping gene RPLP0 were quantified and used as an internal standard to calculate the relative expression of the target genes with respect to RPLP0. Dunnett's multiple comparison test was performed on obtained measurements, and measurements with a significance level of a one-sided 5% compared with the control were deemed to be significantly effective.

Fig. 3 shows relative amounts of gene expression of the Bmal1 gene at 2 and 16 hours after addition of a variety of test substances and cortisol or forskolin as positive controls.

Table 1 below shows relative amounts of gene expression of the Bmal1 gene at 16 hours. For some test substances, relative amounts of gene expression of the Has2 gene were also shown in Table 1.

**[Table 1]**

| Test substance | Relative amount of Bmal1 gene expression (16 hours) | Relative amount of Has2 gene expression (16 hours) |
|---|---|---|
| Control | 0.41 | 4.6 |

| Herbal medicine | | |
|---|---|---|
| Hinoki Cypress*** | 0.64** | |
| Chlorella*** | 0.63** | |
| Hop*** | 0.55* | |
| Zanthoxylum*** | 0.6** | |

| Fragrance | | |
|---|---|---|
| Juniper*** | 2.69** | 163 |
| Lavender*** | 1.2** | |
| Eucalyptus*** | 1.73** | 1549** |
| Olibantun*** | 1.09* | |
| Cypress*** | 1.13** | 62.6** |
| Palmarosa*** | 1.07* | |
| Pineneedle*** | 1.26** | |
| Rose*** | 1.01* | |
| Ylang-ylang*** | 4.25** | 66** |
| Elemi | 1.3** | 448** |
| Petitgrain*** | 1.13* | |
| Pepper*** | 1.98** | 78.8** |
| Thyme*** | 1.5** | |
| Camomile*** | 2.25** | |

| | | |
|---|---|---|
| *: p<0.05, **: p<0.01 ***: Comparative Example | | |

It was demonstrated that hinoki cypress extract, chlorella extract, hop extract, Zanthoxylum extract, juniper oil, lavender oil, eucalyptus oil, olibanum, cypress oil, palmarosa oil, pineneedle oil, rose oil, ylang-ylang oil, elemi oil, petitgrain oil, pepper oil, thyme oil, and chamomile oil, of which only elemi oil is according to the invention and all other are comparative examples, as well as positive controls of cortisol and forskolin, can induce an expression rhythm with a peak at 16 hours after stimulation, and significantly increase amounts of gene expression of Bmal1 and, accordingly, that these herbal medicines or fragrances can modulate expression of the Bmal gene.

In addition, it was suggested that juniper, eucalyptus, cypress, ylang-ylang, elemi and pepper, of which only elemi is according to the invention and all other are comparative examples, can improve or augment skin functions by enhancing hyaluronic acid production, as they were shown to promote Has2 gene expression 16 hours after stimulation.

The expression modulator for the Bmal gene for use according to the present invention may be used in combination with an expression modulator for a Period gene, which include, but are not limited to, herbal medicines such as arnica, nuphar, black tea extract, Zanthoxylum, and other extracts; and fragrances such as juniper, cedar, lavender, clove bud, cypress, rose, ylang-ylang, galbanum, petitgrain, pepper, thyme, basil, beta-caryophyllene, and other oils. It has been confirmed that these herbal medicines or fragrances can induce expression rhythm of the Period1 gene or promote its expression, as a result of examining expression rhythm of Period genes in cultured skin fibroblastic cells with Period1 as the representative. Table 2 below shows relative amounts of gene expression of the Period1 gene at 16 hours after stimulation.

**[Table 2]**

| Test substance | Relative amount of Period1 gene expression (2 hours) |
|---|---|
| Control | 0.23 |
| Herbal medicine | |
| Arnica*** | 0.56** |
| Black tea extract*** | 0.48* |
| Nuphar*** | 0.51** |
| Zanthoxylum*** | 0.42* |
| Fragrance | |
| Juniper*** | 0.67* |
| Cedar*** | 0.7* |
| Lavender*** | 0.88** |
| Clove Bud*** | 1.11** |
| Cypress*** | 1.29** |
| Rose*** | 0.75* |
| Ylang-ylang*** | 1.08** |
| Galbanum*** | 0.76* |
| Petitgrain*** | 1.06** |
| Pepper*** | 1.44** |
| Thyme*** | 1.81** |
| Basil*** | 0.68* |
| beta-Caryophyllene*** | 0.44* |

| | |
|---|---|
| *: p<0.05, **: p<0.01 ***: comparative examples | |

### (Compositional examples)

Compositional comparative examples are given below. In the compositional examples below, ylang-ylang oil is used. One, or a mixture of more than one, of the herbal medicines and/or fragrances which are described above as being capable of modulating the expression of the Bmal gene may be contained. Compositional amounts are all represented by mass percent relative to the total amount of each product.

| [Fragrance] | | |
|---|---|---|
| (1) | Alcohol | 75.0 |
| (2) | Purified water | remainder |
| (3) | Dipropylene glycol | 5.0 |
| (4) | Expression Modulator for Bmal Gene: ylang-ylang oil | 10.0 |
| (5) | Antioxidant | 8.0 |
| (6) | Dye | as needed |
| (7) | UV absorbent | as needed |

| [Room freshener] | | |
|---|---|---|
| (1) | Alcohol | 80.0 |
| (2) | Purified water | remainder |
| (3) | Antioxidant | 5.0 |
| (4) | Expression modulator for Bmal gene (ylang-ylang oil) | 3.0 |
| (5) | 3-methyl-3-methoxybutanol | 5.0 |
| (6) | Dibenzylidene sorbitol | 5.0 |

| [Incense] | | |
|---|---|---|
| (1) | Makko powder | 75.5 |
| (2) | Sodium benzoate | 15.5 |
| (3) | Expression modulator for Bmal gene (ylang-ylang oil) | 5.0 |
| (4) | Eucalyptus oil | 1.0 |
| (5) | Purified water | remainder |

| [Bath salts] | | |
|---|---|---|
| (1) | Sodium sulfate | 45.0 |
| (2) | Sodium bicarbonate | 45.0 |
| (3) | Lavender oil | 9.0 |
| (4) | Expression modulator for Bmal gene (ylang-ylang oil) | 1.0 |

| [Massage gel] | | |
|---|---|---|
| (1) | Erythritol | 2.0 |
| (2) | Caffeine | 5.0 |
| (3) | Phellodendron amurense bark extract | 3.0 |
| (4) | Glycerin | 50.0 |
| (5) | Carboxyvinyl polymer | 0.4 |
| (6) | Polyethylene glycol 400 | 30.0 |
| (7) | Trisodium edetate | 0.1 |
| (8) | Polyoxylene (10) methylpolysiloxane copolymer | 2.0 |
| (9) | Squalane | 1.0 |
| (10) | Potassium hydroxide | 0.15 |
| (11) | Expression modulator for Bmal gene (ylang-ylang oil) | 1.0 |

| [Massage cream] | | |
|---|---|---|
| (1) | Solid paraffin | 5.0 |
| (2) | Beeswax | 10.0 |
| (3) | Vaseline | 15.0 |
| (4) | Fluid paraffin | 41.0 |
| (5) | 1,3-butylene glycol | 4.0 |
| (6) | Glycerin monostearate | 2.0 |
| (7) | POE (20) sorbitan monolaurate ester | 2.0 |
| (8) | Borax | 0.2 |
| (9) | Caffeine | 2.0 |
| (10) | Preservative | as needed |
| (11) | Antioxidant | as needed |
| (12) | Expression modulator for Bmal gene (ylang-ylang oil) | 1.0 |
| (13) | Purified water | remainder |

### [Aromatic fiber]

Microcapsules containing the expression modulator for Bmal gene (particle diameter: no greater than 50 µm (micrometer); percentage of essential oil in microcapsule: 50 wt%) were added to a cuproammonium cellulose solution (cellulose concentration: 10 wt%; ammonium concentration: 7 wt%; copper concentration 3.6 wt%) in the range of 0.1 wt% to 20 wt% of the cellulose weight, mixed, and spun by a conventional wet spinning method, and aromatic fiber was obtained following a refining step and a drying step.

| [Granules] | | |
|---|---|---|
| (1) | Sucralose | 0.1 |
| (2) | Expression modulator for Bmal gene (ylang-ylang oil) | 0.1 |
| (3) | Flavoring | 5.0 |
| (4) | Excipient (Ceolus) | 10.0 |
| (5) | Maltitol | remainder |

| [Tablets (chewable type)] | | |
|---|---|---|
| (1) | Inositol | 11.0 |
| (2) | Maltitol | 21.0 |
| (3) | Sucrose | 0.5 |
| (4) | Salmon sperm extract (DNA Na) | 0.1 |
| (5) | Yeast extract | 0.1 |
| (6) | Expression modulator for Bmal gene (ylang-ylang oil) | 0.1 |
| (7) | Flavoring | 5.0 |
| (8) | Excipient | remainder |

| [Tablets] | | |
|---|---|---|
| (1) | Lubricant (sucrose fatty acid ester, etc.) | 1.0 |
| (2) | Gum arabic aqueous solution (5%) | 2.0 |
| (3) | Acidulant | 1.0 |
| (4) | Colorant | as needed |
| (5) | Expression modulator for Bmal gene (ylang-ylang oil) | 0.1 |
| (6) | Sugars (powdered sugar, sorbitol, etc.) remainder | |

| [Candy] | | |
|---|---|---|
| (1) | Sugar | 50.0 |
| (2) | Starch syrup | 47.95 |
| (3) | Organic acids | 2.0 |
| (4) | Expression modulator for Bmal gene (ylang-ylang oil) | 0.05 |

| [Gum] | | |
|---|---|---|
| (1) | Sugar | 43.0 |
| (2) | Gum base | 30.95 |
| (3) | Glucose | 10.0 |
| (4) | Starch syrup | 16.0 |
| (5) | Expression modulator for Bmal gene (ylang-ylang oil) | 0.05 |

Products of these compositional examples can regulate expression of Bmal gene and modulate the circadian rhythm of the living organism by a trial use of each product form in a typical manner of use.

## Claims

1. Cosmetic use of Elemi oil for modulating the circadian rhythm for improving rough skin resulting from the malfunction of the circadian rhythm by inducing expression rhythms of a Bmal gene or promoting expression of a Bmal gene.

2. Cosmetic use of Elemi oil according to claim 1, wherein Elemi oil is provided as external formulation to be applied on the skin.

3. Cosmetic use of Elemi oil according to claim 2, wherein the external formulation includes perfume, eaux de toilet, eaux de cologne, cream, emulsion, foundation, face powder, lip stick, soap, shampoo and conditioner, body shampoo, body rinse, body powder, and bath soap.

4. Cosmetic use of Elemi oil according to claim 2 or 3, wherein the external formulation is provided in the dosage form of a water-based system, solubilized system, emulsion, oilbased system, gel, paste, ointment, aerosol, water-oil two-phase system, or water-oil-powder three phase system.

## Patentansprüche

1. Kosmetische Verwendung von Elemi-Öl zur Modulierung des Biorhythmus zur Verbesserung von durch Fehlfunktion des Biorhythmus verursachter rauer Haut durch Induzieren von Expressionsrhythmen eines Bmal-Gens oder Fördern der Expression eines Bmal-Gens.

2. Kosmetische Verwendung von Elemi-Öl nach Anspruch 1, wobei Elemi-Öl als äußere Formulierung zur Auftragung auf der Haut bereitgestellt wird.

3. Kosmetische Verwendung von Elemi-Öl nach Anspruch 2, wobei die äußere Formulierung Parfüm, Eaux de Toilet, Kölnisch Wasser, Creme, Emulsion, Foundation, Gesichtspuder, Lippenstift, Seife, Shampoo und Spülung, Körpershampoo, Körperspülung, Körperpuder und Badeseife einschließt.

4. Kosmetische Verwendung von Elemi-Öl nach Anspruch 2 oder 3, wobei die äußere Formulierung in der Darreichungsform eines Wasser-basierenden Systems, eines gelösten Systems, einer Emulsion, eines Öl-basierenden Systems, eines Gels, einer Paste, einer Salbe, eines Aerosols, eines Wasser-Öl-Zweiphasensystems oder eines Wasser-Öl-Pulver-Dreiphasensystems bereitgestellt wird.

## Revendications

1. Utilisation cosmétique d'huile d'Elemi pour moduler le rythme circadien afin d'améliorer la peau rêche résultant du dysfonctionnement du rythme circadien par induction de rythmes d'expression du gène Bmal ou par promotion de l'expression d'un gène Bmal.

2. Utilisation cosmétique d'huile d'Elemi selon la revendication 1, dans laquelle de l'huile d'Elemi est pourvue en tant que formulation externe pour être appliquée sur la peau.

3. Utilisation cosmétique d'huile d'Elemi selon la revendication 2, dans laquelle la formulation externe comprend du parfum, de l'eau de toilette, de l'eau de cologne, de la crème, de l'émulsion, de la fondation, de la poudre de visage, du stick à lèvres, du savon, du shampooing et du conditionneur, du shampooing de corps, du rinçage de corps, de la poudre de corps, et du savon de bain.

4. Utilisation cosmétique d'huile d'Elemi selon la revendication 2 ou 3, dans laquelle la formulation externe est pourvue sous la forme de dosage d'un système sur base d'eau, d'un système solubilisé, d'une émulsion, d'un système à base d'huile, d'un gel, d'une pâte, d'un baume, d'un aérosol, d'un système à deux phases eau-huile, ou d'un système à trois phases eau-huile-poudre.
